(19) Europäisches Patentamt
European Patent Office
Office européen des brevets

(11) Veröffentlichungsnummer: **0 359 048**
**A2**

# (12) EUROPÄISCHE PATENTANMELDUNG

(21) Anmeldenummer: **89116090.5**

(22) Anmeldetag: **31.08.89**

(51) Int. Cl.5: **C07C 235/76 , C07C 233/36 , C07C 233/37 , C07C 69/593 , C07C 57/13 , C23F 11/12 , C23F 11/14**

Patentansprüche für folgenden Vertragsstaat: ES.

(30) Priorität: **03.09.88 DE 3830068**

(43) Veröffentlichungstag der Anmeldung: **21.03.90 Patentblatt 90/12**

(84) Benannte Vertragsstaaten: **BE DE ES FR GB IT NL**

(71) Anmelder: **HOECHST AKTIENGESELLSCHAFT**
**Postfach 80 03 20**
**D-6230 Frankfurt am Main 80(DE)**

(72) Erfinder: **Ritschel, Werner, Dr.**
**Kohlweg 11**
**D-6240 Königstein/Taunus(DE)**
Erfinder: **Mitzlaff, Michael, Dr.**
**Brüningstrasse 33a**
**D-6380 Bad Homburg(DE)**
Erfinder: **Hoffmann, Hermann, Dr.**
**In den Padenwiesen 13**
**D-6233 Kelkheim (Taunus)(DE)**

(54) **Amindoamin-Salze von Alkenylbernsteinsäurederivaten, Verfahren zu deren Herstellung und deren Verwendung als Korrosionsinhibitoren.**

(57) Amidoamin-Salze von Alkenylbernsteinsäurederivaten der Formeln

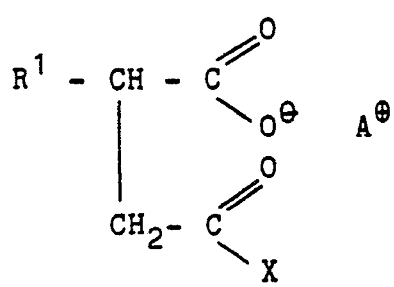

(Ia)

bzw.

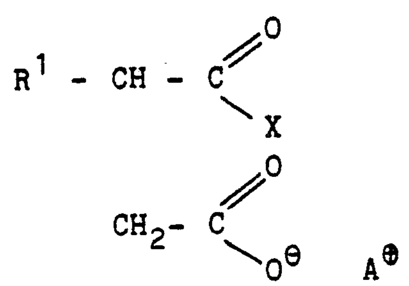

(Ib)

wobei $R^1$ $C_6$-$C_{22}$, vorzugsweise $C_9$-$C_{18}$-Alkenyl,
X eine Gruppe der Formel OH, $OR^2$, $NH\text{-}R^2$ oder $NR^2_2$,
$R^2$ $C_1$-$C_{18}$-Alkyl oder Cycloalkyl,
A ein Amidoamin der Formel II

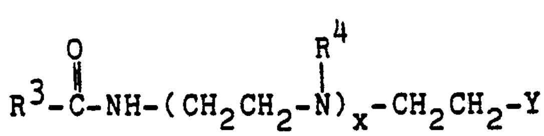

(II)

EP 0 359 048 A2

in protonierter Form,
$R^3$ $C_5$-$C_{22}$-, vorzugsweise $C_{10}$-$C_{18}$-Alkyl, $C_5$-$C_{22}$-, vorzugsweise $C_{10}$-$C_{18}$-Alkenyl oder Cycloalkyl,
$R^4$ eine Gruppe der Formel $-(CH_2-\underset{Z}{\underset{|}{C}}HO)_nH$,

Y eine Gruppe der Formeln $-NH-\underset{O}{\underset{\|}{C}}-R^3$, $-O-\underset{O}{\underset{\|}{C}}-R^3$,

$-(CH_2-\underset{Z}{\underset{|}{C}}HO)_nH$

OH oder $NH_2$, Z Wasserstoff oder Methyl, n eine Zahl von 0 bis 12 und x eine Zahl von 1 bis 3 bedeuten.
Die genannten Salze werden eingesetzt als Korrosionsinhibitoren bei Öl-Wasser Emulsionne, besonders bei Erdöl.

# Amidoamin-Salze von Alkenylberensteinsäurederivaten, Verfahren zu deren Herstellung und deren Verwendung als Korrosionsinhibitoren

Korrosionsprobleme treten bei allen Prozessen der Erdölgewinnung und -verarbeitung auf, bei denen Eisen oder Eisen enthaltende Metalle mit wäßrigen Systemen in Berührung kommen. Besonders gravierend sind die Probleme bei der Einwirkung von Salzwasser, Kohlensäure und Schwefelwasserstoff.

Die Schutzwirkung der bekannten Handelsprodukte - meist werden Amine oder quaternäre Ammoniumverbindungen eingesetzt - ist aber häufig unzureichend oder verschlechtert sich nach kurzer Zeit, da die Verbindungen durch mechanische Einflüsse oder Reaktionen mit aggressiven Medien von der zu schützenden Metalloberfläche wieder abgelöst werden. Allein durch häufiges Nachdosieren kann das Problem nur selten gelöst werden. Insbesondere dann, wenn sich die Zusammensetzung von frisch gefördertem Rohöl laufend ändert, z.B. wenn zur Erhöhung der Feldausbeute mit Salzwasser geflutet wird, ist die Gefahr der Schädigung der Anlagen durch Korrosion sehr hoch, so daß die Forderung nach neuen, besseren Korrosionsschutzmitteln mit Langzeitwirkung gestellt wird.

Es wurde nun gefunden, daß durch Verwendung der nachfolgend beschriebenen Amidoamin-Salze von Alkenylbernsteinsäurederivaten eine hervorragende Korrosionsschutzwirkung für Wasser/Öl-Emulsionen, wie sie beim Erdöl vorliegen, erreicht wird, wobei insbesondere die Langzeitwirkung im Vergleich zu herkömmlichen Korrosionsinhibitoren verbessert wird.

Verschiedene Bernsteinsäurederivate sind schon als wasserlösliche Korrosionsinhibitoren bekannt, wobei entweder die freien Säuren oder deren Mono-, Di- oder Triethanolamin-Salze eingesetzt werden. Beispielsweise werden Alkyl- oder Alkenylbernsteinsäurehalbester im US Pat. 4 053 426 beschrieben, die als Triethanolamin-Salz verwendet werden. Waserlösliche Alkanolaminsalze von Alkenylbernsteinsäurehalbamiden für Metallbearbeitungsflüssigkeiten werden beschrieben in US 4 609 531, US 4 729 841, US 4 724 124, DE 3 300 874, US 4 705 666.

Als öllösliche Verbindungen sind im US-Patent 4 235 874 Alkenylbernsteinsäure oder -anhydrid und Triester von Triethanolamin als Korrosionsinhibitoren für raffinierte Erdöl-Produkte genannt. Für die gleichen Anwendungsgebiete werden im US-Patent 4 148 605 Ester-dicarbonsäuren beschrieben, die durch Umsetzung von Alkenylbernsteinsäureanhydrid mit Hydroxy-carbonsäuren erhalten werden; im US-Patent 4 326 987 werden Umsetzungsprodukte aus Alkenylbernsteinsäureanhydrid mit Ether-diaminen genannt. Die US-Patentschrift 2 490 794 nennt Umsetzungsprodukt von Alkenylbernsteinsäureanhydriden mit primären Aminen im Molverhältnis 1,25 bis 2:1 als Rostschutzmittel in Schmieröle. Weiterhin bekannt als öllösliche Verbindungen sind Umsetzungsprodukte von Alkenylbernsteinsäureanhydriden mit langkettigen Dialkylaminen, z.B. Ditalgamin, durch US-PS 3 646 151.

Für die Anwendung in Benzin/Alkohol-Mischungen werden in US 4 737 159 Kombinationen von Alkenylbernsteinsäuren mit aliphatischen oder cycloaliphatischen Aminen als Korrosionsinhibitoren beschrieben, die zusätzlich Detergentien, z.B. acylierte Polyamine, enthalten können.

Alle diese vorweg beschriebenen Verbindungen bzw. Umsetzungsprodukte weisen in korrosiven Wasser-in-Öl-Emulsionen aber ungenügende Korrosionsschutzwirkungen auf, weil die Verbindungen entweder ungenügende Öllöslichkeit besitzen oder aber aufgrund ihrer chemischen Strukturen unzureichende Inhibitorwirkung entfalten.

Bestimmte, gut wirksame Amidoamin-Salze von Alkenylbernsteinsäurehalbamiden sind bereits in US 4 722 812 beschrieben. Die dort genannten, am Stickstoff unsubstituierten Alkenylbernsteinsäurehalbamide erfordern aber ein aufwendiges Hantieren mit flüssigem oder gasförmigen Ammoniak, was bei den nachfolgend beschriebenen Verbindungen vermieden wird.

Wie im folgenden erläutert, zeigen Amidoamin-Salze von verschiedenen Alkenylbernsteinsäurederivaten sowohl gute Löslichkeit in öligen Systemen als auch hervorragende Korrosionsschutzeigenschaften, wobei die Wirkung der Einzelkomponenten (d.h. die Alkenylbernsteinsäurederivate bzw. die Amidoamine) bei weitem übertroffen wird.

Gegenstand der Erfindung sind Amidoamin-Salze von Alkenylbernsteinsäurederivaten der Formeln

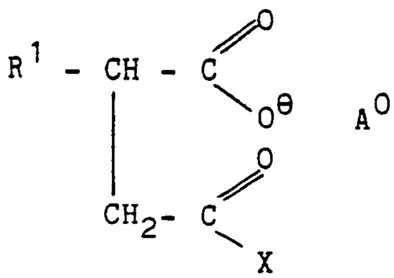

bzw.

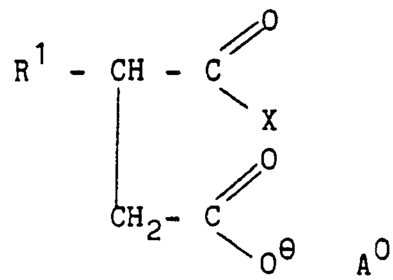

(Ia) (Ib)

wobei R' $C_6$-$C_{22}$, vorzugsweise $C_9$-$C_{18}$-Alkenyl,
X eine Gruppe der Formel OH, $OR^2$, NH-$R^2$ oder $NR^2_2$,
$R^2$ $C_1$-$C_{18}$-Alkyl oder Cycloalkyl,
A ein Amidoamin der Formel II

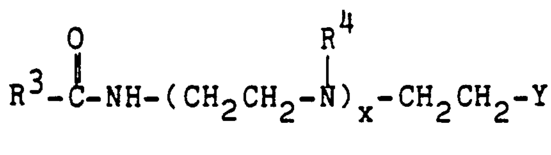

(II)

in protonierter Form,
$R^3$ $C_5$-$C_{22}$-, vorzugsweise $C_{10}$-$C_{18}$-Alkyl, $C_5$-$C_{22}$-, vorzugsweise $C_{10}$-$C_{18}$-Alkenyl oder Cycloalkyl,
$R^4$ eine Gruppe der Formel

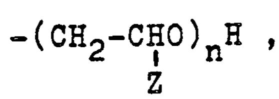

Y eine Gruppe der Formeln $-NH-\underset{\overset{\|}{O}}{C}-R^3$, $-O-\underset{\overset{\|}{O}}{C}-R^3$, $-(CH_2-\underset{\overset{|}{Z}}{C}HO)_nH$ OH oder $NH_2$, Z Wasserstoff oder Methyl, n eine Zahl von 0 bis 12 und x eine Zahl von 1 bis 3 bedeuten. Cycloalkyl unter $R^3$ bedeutet vorzugsweise solche Gruppen, die sich von den Naphthensäuren ableiten.

Die Herstellung der Salze der Formeln Ia und Ib erfolgt in der Weise, daß zunächst ein Alkenylbernsteinsäureanhydrid mit Wasser, einem Alkohol oder einem Amin zur Alkenylbernsteinsäure, einem Alkenylbernsteinsäure-halbester oder einem Alkenylbernsteinsäure-halbamid der folgenden Formeln umgesetzt wird:

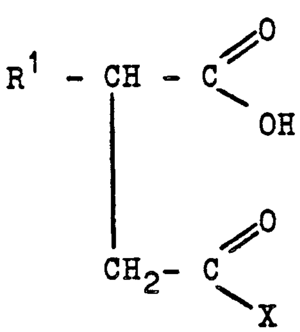

bzw.

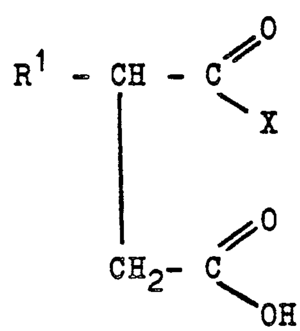

Aus diesen Alkenylbernsteinsäuren-derivaten werden dann die erfindungsgemäßen Salze hergestellt durch Neutralisation mit einem Amidoamin der Formel II. Die Amidamine werden hergestellt durch Amidierung von Carbonsäuren mit Aminen wie Diethylentriamin, Triethylentetramin, Tetraethylenpentamin oder Aminoethylethanolamin bei ca. 150-160 °C und gegebenenfalls anschließender Oxalkylierung mit Ethylenoxid oder Propylenoxid unter üblicherweise bekannten Reaktionsbedingungen.

Bei der Amidierungsreaktion wird Wasser abdestilliert; durch Verwendung von hohen Temperaturen

(über 160°C) oder Vakuum können dabei auch Imidazoline entstehen, die die gleiche Wirksamkeit wie die Amidoamine aufweisen. Höhere Reaktionstemperaturen sind daher nicht schädlich, aber auch nicht erforderlich.

Vor der Neutralisation verdünnt man die Alkenylbernsteinsäurederivate zweckmäßigerweise mit einem geeigneten Lösungsmittel, beispielsweise mit einem niederen Alkohol oder einem dünnflüssigen Öl und stellt damit eine Konzentration von ca. 30 bis 50% an Wirksubstanz ein. Durch Zugabe dieser Handelsware in Mengen von 5 bis 100, vorzugsweise 10 bis 50 ppm zur Erdöl oder Erdölprodukten erhält man die gewünschte Korrosionsschutzwirkung.

**Allgemeine Angaben zur Herstellung der Alkenylbernsteinsäurederivate für die nachfolgenden**

**Beispiele**

**Alkenylbernsteinsäuren** werden hergestellt durch Reaktion von Alkenylbernsteinsäureanhydrid mit Wasser in 2 Stunden bei 100°C.

**Alkenylbernsteinsäureanhalbester** werden hergestellt durch Umsetzung von 1 Mol Alkenylbernsteinsäureanhydrid mit mindestens 1 Mol Alkohol durch 5-stündiges Erhitzen auf 80 bis 120°C.

**Alkenylbernsteinsäurehalbamide** werden hergestellt durch Umsetzung von 1 Mol Alkenylbernsteinsäureanhydrid mit 1 Mol Amin in 1 Stunde bei 80°C.

**Beispiel 1**

284 g (1 Mol) n-Dodecenylbernsteinsäure ($I_1$) werden mit 377 g (1 Mol) Amidoamin $II_1$ (hergestellt aus 292 g (1 Mol) Tallölfettsäure und 103 g (1 Mol) Diethylentriamin in Gegenwart von 643 g Isopropanol 1 Stunde bei 40°C gerührt. Man erhält eine gelbe Lösung mit einem Wirkstoffgehalt von 50 %.

$I_1$:
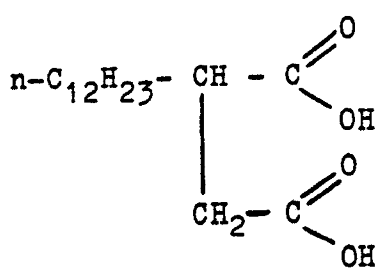

$II_1$:
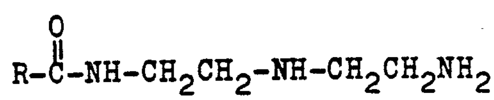

R = Alkylkette der Tallölsäure

**Beispiel 2**

382 g (1 Mol) Octadecenylbernsteinsäure-monomethylester ($I_2$) werden mit 380 g (1 Mol) des Amidoamin $II_2$ (hergestellt aus 1 Mol Naphthensäure und 1 Mol Aminoethylethanolamin) in Gegenwart von 760 g Shellsol AB 1 Stunde bei 40°C gerührt. Man erhält eine klare Lösung mit einem Wirkstoffgehalt von 50 %.

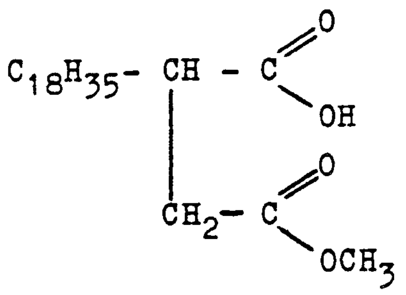

bzw.

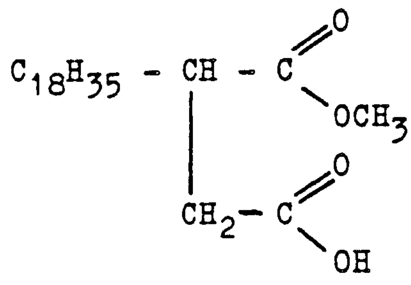

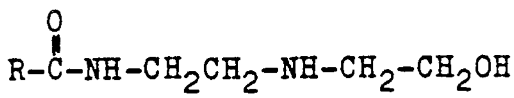

R = Alkylgerüst von Naphthensäure

**Beispiel 3**

421 g (1 Mol) N-Cocosalkyl-tripropenylbernsteinsäurehalbamid ($I_3$) werden mit 739 g (1 Mol) Amidoamin $II_3$ (hergestellt aus 2 Mol Talgfettsäure, 1 Mol Diethylentriamin und 3 Mol Ethylenoxid) in Gegenwart von 1160 g Isobutanol 1 Stunde bei 40 °C gerührt. Man erhält eine braune Lösung mit einem Gehalt von 50 % Wirksubstanz.

$I_3$:

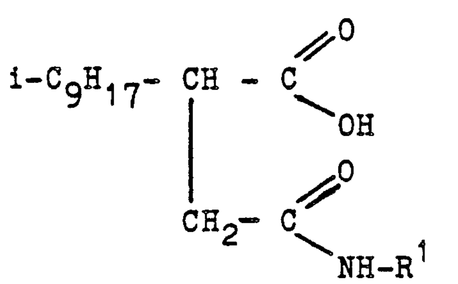

bzw.

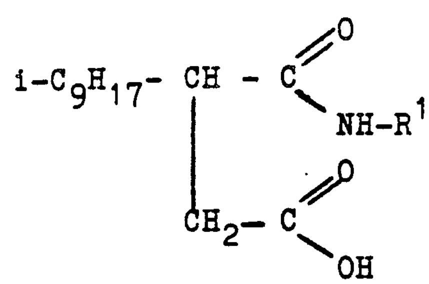

$R^1$ = Cocosalkyl

$II_3$:

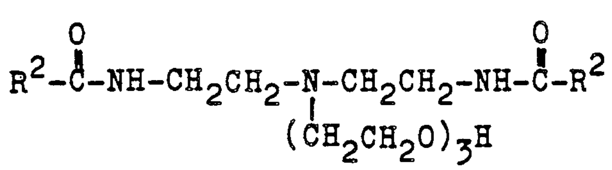

$R^2$ = Alkylkette der Talgfettsäure

**Beispiel 4**

395 g (1 Mol) N,N-Dibutyl-tetrapropenylbernsteinsäurehalbamid ($I_4$) werden mit 1020 g (1 Mol) Amidoamin $II_4$ (hergestellt aus 2 Mol Tallölsäure, 1 Mol Triethylentetramin und 6 Mol Propylenoxid) in Gegenwart von 1400 g Shellsol AB 1 Stunde bei 40 °C gerührt. Man erhält eine braune Lösung mit einem Wirkstoffgehalt von 50 %.

I4: 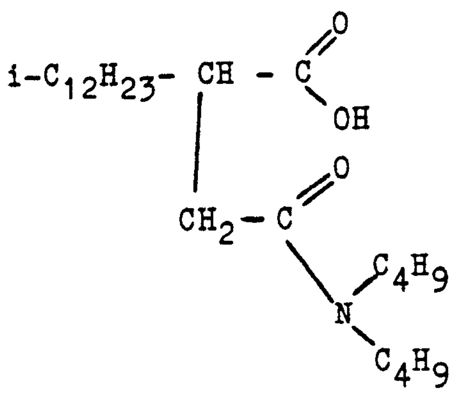 bzw. 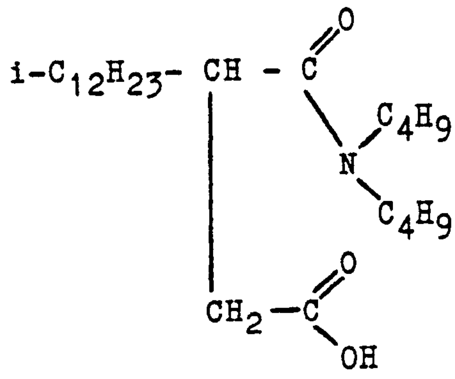

II4: 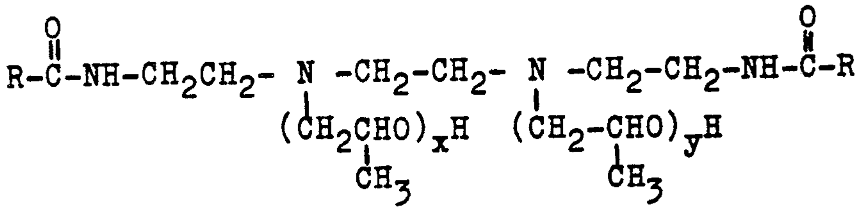

R = Alkylkette der Talgfettsäure x + y = 6

Die nachfolgend beschriebene Ausprüfung zeigt die hervorragenden Korrosionsschutzeigenschaften dieser Verbindungsklasse. Zum Vergleich wurden die Handelsprodukte Visco 938 und Servo CK 378 mitgeprüft.

Zur Prüfung der Inhibitoren wurde ein dynamischer Test (sog. "Wheel-Test") herangezogen sowie eine elektrochemische Methode, die sog. "Elektrochemische Impedanz-Spektroskopie".

Der "Wheel-Test" ist eine gängige Methode für die Testung von Korrosionsinhibitoren für die Erdöl- und Erdgasförderung. Als Testcoupons wurden Stahlbleche der Abmessungen 130 mm x 10 mm x 1 mm gewählt. Diese Blechstreifen wurden geschmirgelt, mit Toluol entfettet und gewogen. Als Testmedium diente Kerosin, das Salzwasser mit 5 Gew.-% NaCl (bezogen auf Wasser) emulgiert enthielt. Die Emulsion enthielt 90 Gew.-% Salzwasser und war mit $H_2S$ bzw. $CO_2$ gesättigt.

Dann wurden 10, 20 und 50 ppm - bezogen auf das Gewicht der Emulsion - an Inhibitor zugesetzt.

Die entfetteten und gewogenen Bleche wurden anschließend in die Emulsionen eingebracht und bei 70 °C 24 Stunden einer mechanischen Bewegung (40 Upm mittels einer die Testgefäße drehenden Welle) unterzogen.

Die Testblechstreifen wurden anschließend mit einer inhibierenden Säure gereinigt, entfettet und nach Trocknung zur Bestimmung des Gewichtsverlustes gewogen. Die Korrosionsraten sind in mpy (mills per year) angegeben (39.4 mpy = 1 mm/Jahr). Zum Vergleich wurde der Blindwert (Versuch ohne Inhibitorzusatz) ermittelt.

Die mit dieser Testmethode erhaltenen Ergebnisse sind in der Tabelle 1 zusammengefaßt.

Tabelle 1:

| Ergebnisse Wheel-Test | | | | | | |
|---|---|---|---|---|---|---|
| 70 °C, 24 h, Salzwasser 5% Kerosin 9:1, Korrosionsraten in mpy | | | | | | |
| | $CO_2$ (pH = 3.5 mit Eisessig) | | | $H_2S$ | | |
| | 10 | 20 | 50 ppm | 10 | 20 | 50 |
| Bsp. 1 | 4,5/3,8 | 2,8/1,4 | 1,4/1,1 | 2,3/1,9 | 2,0/1,7 | 1,0/0,5 |
| Bsp. 2 | 3,9/4,4 | 3,2/3,0 | 2,9/2,5 | 6,4/5,4 | 3,9/4,2 | 2,8/2,2 |
| Bsp. 3 | 5,8/6,0 | 4,2/3,5 | 2,9/3,4 | 9,5/10,6 | 5,7/5,9 | 3,1/2,9 |
| Bsp. 4 | 7,1/6,8 | 4,5/4,1 | 1,9/1,7 | 4,9/5,0 | 4,0/4,1 | 2,2/1,5 |
| Visko 938 | 26,6/25,4 | 18,6/2,0 | 8,4/6,4 | 16,4/19,0 | 8,8/10,4 | 3,6/4,0 |
| Servo CK 378 | 14,0/12,3 | 5,2/4,6 | 3,4/4,5 | 16,0/13,5 | 10,3/10,0 | 3,5/5,0 |
| Blindwert | 25,3 + 6,1 (10 Werte) | | | 51,3 + 4,9 (10 Werte) | | |

Als weitere Testmethode diente die Elektrochemische Impedanz-Spektroskopie. Mit dieser verhältnismäßig neuen Prüfmethode kann u.a. der zeitliche Verlauf der Inhibitorwirkung verfolgt werden, d.h. man kann die Korrosionsschutzwirkung nach 2, 4 oder 24 Stunden überprüfen und so zusätzliche Informationen über Inhibitorwirkung bzw. Vergleiche mit anderen Inhibitoren erhalten.

Aufbau und Wirkungsweise der Elektrochemischen Impedanz-Spektroskopie ist in der Fachliteratur beschrieben, siehe z.B. W.J. Lorenz und F. Mansfeld, Electrochimica Acta, Vol. 31, No. 4, pp 467-476, 1986. Als Maß für die Inhibitorwirkung dient der Polarisationswiderstand $Rp^{ac}$; hohe $Rp^{ac}$-Werte zeigen gute Inhibitorwirkung, niedrige Werte entsprechen geringerer Schutzwirkung, der Blindwert ist der $Rp^{ac}$-Wert ohne Inhibitorzusatz.

Als Meßelektrode dient eine rotierende Schneibenelektrode aus dem Handelsstahl RSt 37.2 mit 0,28 $cm^2$ Oberfläche, die Rotationsfrequenz beträgt 1000 Upm. Als Testmedium wurde die dem Wheel-Test entsprechende Salzlösung verwendet (5 Gew.-% NaCl, pH = 3.5 mit Essigsäure eingestellt), die Lösung wurde mit $CO_2$-Gas entlüftet. Dieser Lösung wurden 10 ppm Inhibitor aus den Beispielen 1 bis 4 zugesetzt, zum Vergleich dienten die Handelsprodukte Visco 938 und Servo CK 378 sowie der Blindwert. Weiterhin wurden die Einzelkomponenten von Beispiel 1 getrennt vermessen ($I_1$ und $II_1$).

Die Tabelle 2 zeigt die ermittelten $Rp^{ac}$-Wete nach 2, 4 und 24 Stunden bei 25 °C.

Tabelle 2:

| Inhibitor | $Rp^{ac}$ (Ohm•$cm^2$) | | |
|---|---|---|---|
| | 2 h | 4 h | 24 h |
| Blindwert | 800 | 750 | 780 |
| Beispiel 1 | 7780 | 9950 | 10800 |
| Beispiel 2 | 8500 | 12300 | 12450 |
| Beispiel 3 | 8430 | 10500 | 11120 |
| Beispiel 4 | 8180 | 11800 | 10740 |
| Visko 938 | 2620 | 2630 | 2570 |
| Servo CK 378 | 2035 | 2010 | 1990 |
| $I_1$ | 700 | 690 | 710 |
| $II_2$ | 6900 | 6850 | 7050 |
| Polarisations-Widerstandsmessungen mit Inhibitoren und Vergleichsmessungen | | | |

Man erkannt aus der Tabelle 2 die hohen Inhibitor-Schutzwerte der Beispiele 1 bis 4 im Vergleich zum Blindwert und Vergleichsinhibitoren. Weiter erkennt man, daß die Schutzwirkung über mehrere Stunden zunimmt, während die Wirkung der Vergleichsprodukte abnimmt. Weiterhin erkennt man, daß die Einzelkomponenten aus Beispiel 1, getrennt geprüft, bei weitem nicht die gute Schutzwirkung erreichen wie die nach dem Patentanspruch formulierten und in den Beispielen beschriebenen Additive.

## Ansprüche

1. Amidoamin-Salze von Alkenylbernsteinsäurederivaten der Formeln

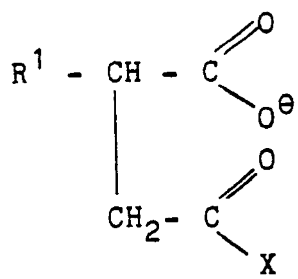

(Ia)

bzw.

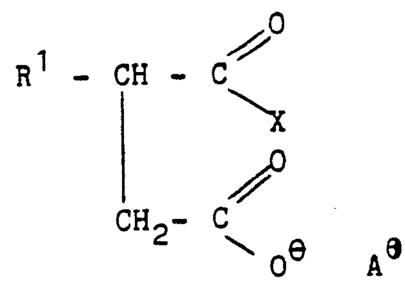

(Ib)

wobei $R^1$ $C_6$-$C_{22}$, vorzugsweise $C_9$-$C_{18}$-Alkenyl,
X eine Gruppe der Formel OH, $OR^2$, NH-$R^2$ oder $NR^2_2$,
$R^2$ $C_1$-$C_{18}$-Alkyl oder Cycloalkyl,
A ein Amidoamin der Formel II

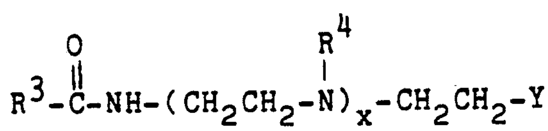

(II)

in protonierter Form,
$R^3$ $C_5$-$C_{22}$-, vorzugsweise, $C_{10}$-$C_{18}$-Alkyl, $C_5$-$C_{22}$-, vorzugsweise $C_{10}$-$C_{18}$-Alkenyl oder Cycloalkyl,
$R^4$ eine Gruppe der Formel $-(CH_2-\underset{Z}{C}HO)_nH$,
Y eine Gruppe der Formeln $-NH-\underset{O}{\overset{}{C}}-R^3$, $-O-\underset{O}{C}-R^3$, $-(CH_2-\underset{Z}{C}HO)_nH$
OH oder $NH_2$, Z Wasserstoff oder Methyl, n eine Zahl von 0 bis 12 und x eine Zahl von 1 bis 3 bedeuten.

2. Verfahren zur Herstellung der Verbindungen nach Anspruch 1, dadurch gekennzeichnet, daß man ein Alkenylbernsteinsäurederivat der Formel

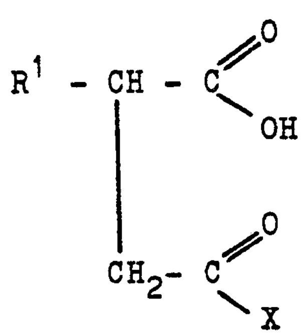

bzw.

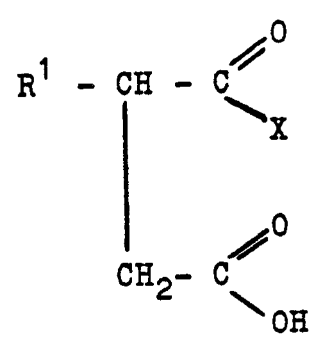

mit einem Amidoamin der Formel

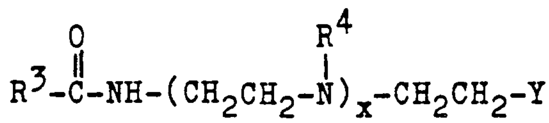

umsetzt.

3. Verwendung der Verbindungen nach Anspruch 1 als Korrosionsinhibitoren bei Öl-Wasser-Emulsionen, insbesondere bei Erdöl.

Patentansprüche für folgenden Vertragsstaat: ES

1. Verfahren zur Herstellung von Amidoamin-Salzen von Alkenylbernsteinsäurederivaten der Formeln

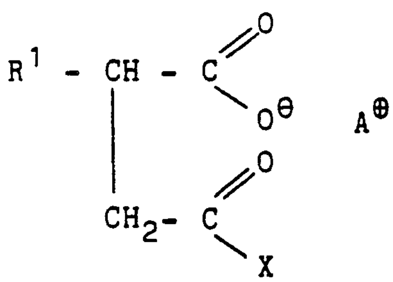

bzw.

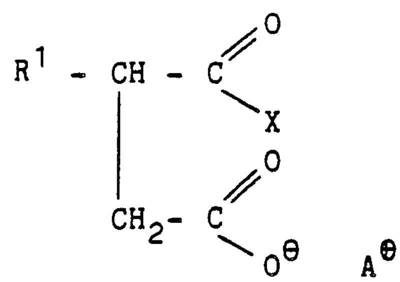

(Ia) (Ib)

wobei $R^1$ $C_6$-$C_{22}$, vorzugsweise $C_9$-$C_{18}$-Alkenyl,
X eine Gruppe der Formel OH, $OR^2$, NH-$R^2$ oder $NR^2_2$,
$R^2$ $C_1$-$C_{18}$-Alkyl oder Cycloalkyl,
A ein Amidoamin der Formel II

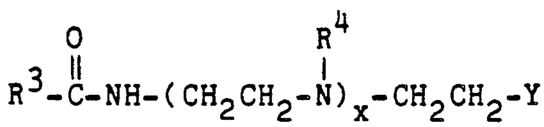

(II)

in protonierter Form,
$R^3$ $C_5$-$C_{22}$-, vorzugsweise $C_{10}$-$C_{18}$-Alkyl, $C_5$-$C_{22}$-, vorzugsweise $C_{10}$-$C_{18}$ Alkenyl oder Cycloalkyl,
$R^4$ eine Gruppe der Formel $-(CH_2-\underset{Z}{C}HO)_nH$,
Y eine Gruppe der Formeln $-NH-\underset{O}{\overset{}{C}}-R^3$, $-O-\underset{O}{C}-R^3$,
$-(CH_2-\underset{Z}{C}HO)_nH$
OH oder $NH_2$, Z Wasserstoff oder Methyl, n eine Zahl von 0 bis 12 und x eine Zahl von 1 bis 3 bedeuten, dadurch gekennzeichnet, daß man ein Alkenylbernsteinsäurederivat der Formel

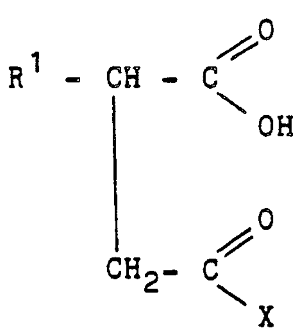

bzw.

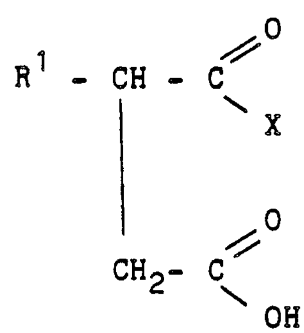

mit einem Amidoamin der Formel

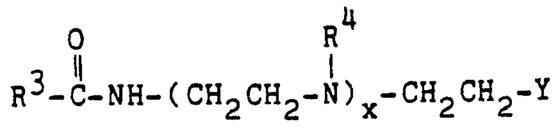

umsetzt.

2. Verwendung der Verbindungen nach Anspruch 1 als Korrosionsinhibitoren bei Öl-Wasser-Emulsionen, insbesondere bei Erdöl.